# FASCICULE DE BREVET EUROPEEN

(11) **EP 0 703 731 B1**
(45) Date de publication et mention de la délivrance du brevet: **08.04.1998**
(21) Numéro de dépôt: 94919731.3
(22) Date de dépôt: 17.06.1994
(51) Int. Cl.: A23L 1/305, A61K 31/195

(54) **COMPOSITION A USAGE ALIMENTAIRE ET/OU PHARMACEUTIQUE PAUVRE EN POLYAMINES ET APPLICATIONS THERAPEUTIQUES**
POLYARMINEARME NÄHR- UND/ODER HEILMITTELZUSAMMENSETZUNG UND THERAPEUTISCHE VERWENDUNG DERSELBEN
FOOD AND/OR PHARMACEUTICAL COMPOSITION HAVING A LOW POLYAMINE CONTENT, AND THERAPEUTICAL USES THEREOF

(30) Priorité: 17.06.1993 FR 9307586; 03.12.1993 FR 9314761
(43) Date de publication de la demande: 03.04.1996
(73) Titulaire: UNIVERSITE DE RENNES I, F-35000 Rennes (FR)
(72) Inventeur: MOULINOUX, Jacques-Philippe, F-35000 Rennes (FR); QUEMENER, Véronique, F-35700 Rennes (FR)
(74) Mandataire: Vidon, Patrice
(86) Numéro de dépôt international: FR9400736
(87) Numéro de publication internationale: WO9500041

(56) Documents cités:
- EP-A- 0 116 693
- EP-A- 0 162 413
- EP-A- 0 248 217
- US-A- 4 112 123
- US-A- 4 201 788
- US-A- 4 207 315
- US-A- 4 859 452
- CELLULAR AND MOLECULAR BIOLOGY, vol.37, no.8, 1991, UK pages 773 - 783 J.P.MOULINOUX ET AL. 'Biological significance of circulating polyamines in oncology' cité dans la demande
- ANTICANCER RESEARCH, vol.11, no.1, Février 1991 pages 175 - 180 J.P.MOULINOUX ET AL. 'Inhibition of the growth of U-251 human glioblastoma in nude mice by polyamine deprivation' cité dans la demande
- ANTICANCER RESEARCH, vol.12, 1992 pages 1447 - 1454 V.QUEMENER ET AL. 'Polyamine deprivation enhances antitumoral efficacy of chemotherapy' cité dans la demande
- CANCER RESEARCH, vol.50, Août 1990 pages 5077 - 5083 N.SEILER ET AL. 'Endogenous and exogenous polyamines in support of tumor growth' cité dans la demande
- S.Kergozien et al., "Polyamine deprivation provokes an antalgic effect", Life Sciences, vol. 58, no. 24. pp 2209-2215, 1996

## Description

L'invention concerne à la fois le domaine alimentaire et le domaine pharmaceutique.

Les polyamines et tout particulièrement la putrescine (I), la spermidine (II) et la spermine (III) sont présentes dans toutes les cellules.

⁺NH₃ - (CH₂)₄ - NH₃⁺ (I)

⁺NH₃ - (CH₂)₃ - NH - (CH₂)₄ - NH₃⁺ (II)

⁺NH₃ - (CH₂)₃ - NH - (CH₂)₄ - NH - (CH₂)₃ - NH₃⁺ (III)

Bien que ces molécules aient été longtemps considérées comme dénuées de tout rôle physiologique et ne représentant qu'une étape terminale du catabotisme tissulaire, de nombreux travaux scientifiques ont montré que les polyamines issues de la décarboxylation de l'ornithine étaient en fait des molécules biologiquement actives et capables d'intervenir à différents niveaux importants de la vie de la cellule.

Ces molécules que l'on trouve non seulement à l'intérieur même des cellules mais également à l'état circulant dans les liquides biologiques de l'organisme, tels que le sang sont issues de trois sources principales :
- la prolifération cellulaire physiologique (croissance et/ou renouvellement des cellules constitutives de l'organisme) et tumorale,
- l'alimentation,
- les bactéries intestinales.

Les polyamines intracellulaires ne proviennent pas simplement d'échanges transmembranaires passifs avec les liquides biologiques de l'organisme mais résultent d'un métabolisme finement régulé et en relation avec le cycle cellulaire. Un accroissement de l'activité de l'enzyme responsable de la voie anabolique intracellulaire principale des polyamines, l'ornithine-décarboxylase (ODC), et donc des concentrations en putrescine, spermidine et spermine est en effet observé en fin de phase G1 et début de phase S, et en début de phase G2 du cycle de réplication cellulaire. L'implication du métabolisme des polyamines dans la réplication cellulaire et donc dans les processus prolifératifs font de ce métabolisme l'une des cibles privilégiées des drogues antiprolifératives, en même temps que la source de nouveaux signaux circulants susceptibles de révéler l'existence d'un processus néoplasique au sein d'un organisme.

Il a été constaté que la croissance tumorale s'accompagne en règle générale d'un accroissement très significatif de l'anabolisme intracellulaire des polyamines qui se retrouvent alors en quantité augmentée dans les liquides biologiques. Bien que le rôle exact des polyamines circulantes en tant que paramètres impliqués dans la régulation (ou la dysrégulation) homéostatique de la prolifération cellulaire ne soit pas élucidé, il a été prouvé que les taux érythrocytaires de polyamines pouvaient être considérés et utilisés comme des marqueurs du niveau hyperplasique tumoral (voir "Biological significance of circulating polyamines in oncology" - Moulinoux, J.-Ph, Quemener, V. et Khan, N.A. - *Cellular and molecular biology* -37(8), 773-783, 1991). Ces travaux ont par ailleurs montré que les polyamines circulantes, provenant soit des cellules elles-mêmes soit du tractus gastro-intestinal (alimentation, bactéries intestinales) participaient au maintien de l'état prolifératif malin.

On connaît dans l'état de la technique divers inhibiteurs de l'ornithine-décarboxylase et notamment des inhibiteurs sélectifs irréversibles de cette enzyme tels que l'α-difluorométhylornithine (α-DFMO) commercialisée par la société Marion Merrell Dow sous la dénomination Eflornithine*™*. Toutefois, l'administration isolée d'α-DFMO en l'absence de tout autre traitement à des patients ou à des animaux cancéreux, s'est révélée inefficace vis-à-vis de la progression tumorale maligne, ceci alors même que cet inhibiteur de la synthèse des polyamines, tout en diminuant de manière très significative les taux intracellulaires de putrescine et de spermidine, inhibait totalement la prolifération cellulaire cancéreuse *in vitro*. L'incapacité de l'α-DFMO à réduire *in vivo* la prolifération cellulaire maligne est très probablement en relation avec le fait que, dans l'organisme, les cellules cancéreuses restaurent la quantité intracellulaire de polyamines nécessaire au maintien de leur prolifération en captant les polyamines présentes dans le milieu extracellulaire, c'est-à-dire dans les différents liquides biologiques dont le sang. Cette hypothèse a été confortée par les travaux de Person et al (*Cancer Research*, 1988, n°48, pp. 4807 à 4811) qui ont montré qu'à la différence de ce que l'on constatait avec des cellules sauvages L1210, des souris inoculées avec des cellules L1210 mutées, rendues incapables de capter les polyamines extracellulaires, répondaient favorablement à un traitement inhibiteur de la biosynthèse de polyamines intracellulaires par l'α-DFMO.

Différents travaux ont par ailleurs mis en évidence que, chez l'animal, l'administration conjointe :
- d'une alimentation dépourvue de polyamines,
- d'α-DFMO,
- d'un inhibiteur de la polyamine-oxydase (PAO) supprimant la rétroconversion oxydative de la spermidine et de la spermine en putrescine, et
- de néomycine et de métronidazole,
entraîne une inhibition quasi-totale de la progression tumorale du carcinome pulmonaire de Lewis 3LL (Seiler N. et al, *Cancer Research*, 1990, n°50, pp. 5077-5083), du glioblastome humain U251 (Moulinoux J-Ph. et al, *Anticancer Research*, 1991, n°11, pp. 175-180), de l'adénocarcinome de la prostate Dunning MAT-LyLu (Moulinoux J-Ph. et al, *Journal of Urology*, 1991, n° 146, pp. 1408,1412) et du neuroblastome humain neuro 2a (Quemener et al, "Polyamines in the gastro-intestinal tract", Dowling R.H., Fölsch I.R. et Löser C Ed., *Kluwer Academic Publishers Boston*, 1992, pp. 375-385).

Il a par ailleurs été également démontré chez l'animal que la déplétion en polyamines pouvait considérablement potentialiser les effets antiprolifératifs des drogues antitumorales conventionnelles (méthotrexate, cyclophosphamide, vindesine) tout en allongeant le temps de survie des animaux et pouvait permettre de réduire les quantités de drogues administrées tout en conservant le même effet antitumoral (Quemener V. et al, "Polyamine deprivation enhances antitumoral efficacy of chemotherapy", *Anticancer Research* n°12, 1992, pp. 1447-1454).

La demande de brevet française n° 93 07586 déposée le 17 juin 1993, dont la Demanderesse demande à bénéficier de la date conformément aux dispositions de l'article L 612.3 du Code de la Propriété Intellectuelle, propose une composition administrable à l'homme à titre d' aliment, de complément alimentaire et/ou de produit de nutrition thérapeutique, permettant de pallier l'inefficacité des traitements réalisés jusqu'ici chez l'homme par administration isolée d'α-DFMO et permettant d'obtenir l'inhibition de la prolifération des cellules tumorales, constituée d'un mélange nutritif pauvre en polyamines contenant moins de 50 picomoles/g de putrescine, de spermidine, de spermine et de cadavérine, comprenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

De récents travaux ont montré qu'une telle composition pouvait toutefois renfermer des taux de polyamines plus élevés que ceux précédemment revendiqués, et permettre une réelle synergie des effets provoqués, chez l'homme, par la diminution des apports extérieurs de polyamines et par l'inhibition de la synthèse intracellulaire de ces composés.

D'autres travaux effectués chez le rat ou la souris conduisent à penser qu'une telle composition, mise en oeuvre chez l'homme dans le cadre d'un traitement visant à dépléter l'organisme cancéreux en polyamines en réduisant toutes les voies d'apport exogène et endogène de ces substances, permettait d'obtenir également des effets thérapeutiques autres que l'inhibition de la prolifération des cellules tumorales.

La présente demande concerne donc une composition pouvant être ingérée par l'homme caractérisée en ce qu'elle est constituée d'un mélange nutritif pauvre en polyamines contenant moins de 1600 picomoles/g de polyamines.

Les principales polyamines étant constitués par la putrescine, la spermine, la spermidine et la cadavérine, la composition contient avantageusement moins 400 picomoles/g de putrescine, moins 400 picomoles/g de spermidine, moins 400 picomoles/g de spermine et moins 400 picomoles/g de cadavérine.

Préférentiellement, ladite composition contient moins 400, préférentiellement moins 200, picomoles/g de polyamines.

Avantageusement, cette composition contient moins 100, préférentiellement moins 50, picomoles/g de putrescine, moins 100, préférentiellement moins 50, picomoles/g de spermidine, moins 100, préférentiellement moins 50, picomoles/g de spermine et moins 100, préférentiellement moins 50, picomoles/g de cadavérine. Une telle composition apporte, journellement, au moins 17 fois moins de putrescine, 40 fois moins de cadavérine, 70 fois moins de spermidine et 220 fois de spermine que l'alimentation naturelle humaine la plus pauvre qui soit en teneur de polyamines, mais qui réponde néanmoins aux besoins nutritionnels humains.

Selon une variante de l'invention, la composition comprend en outre, en pourcentage de poids sec par rapport au poids sec total : 10 à 35% de lipides, 8 à 30% de protéines, 35 à 80% de glucides, jusqu'à 10% d'un mélange constitué de vitamines, de minéraux et d'électrolytes.

Une telle composition pourra être présentée sous forme sèche à dissoudre extemporanément dans un véhicule neutre convenant à l'administration orale ou encore entérale, ou sous forme liquide prête à l'emploi. Dans tous les cas, la composition est présentée sous forme stérile.

Une telle composition est particulièrement bien adaptée à l'homme et constitue un substitutif alimentaire qui permet de carencer les patients de façon efficace en polyamines. Une telle composition permet en effet d'alimenter un patient de façon satisfaisante tout en induisant une carence en polyamines, d'une part en inhibant la synthèse intracellulaire de polyamines et d'autre part en diminuant l'apport de polyamines exogènes.

Une telle composition permet d'inhiber fortement la synthèse endogène des polyamines et diminue de façon très importante l'apport en ces composés puisque les différents ingrédients qui la constituent en sont quasiment dépourvus. Afin de diminuer également les apports en polyamines par les bactéries intestinales, cette composition pourra être administrée concomitamment à une décontamination du tractus gastro-intestinal au moyens d'antibiotique(s) et/ou d'antiparasitaire(s), tels que, par exemple, la néomycine et le métronidazole. On pourra d'ailleurs envisager d'inclure de tels antibiotique(s) et/ou antiparasitaire(s) directement dans ladite composition, sans sortir du cadre de l'invention.

Les nutriments utilisés dans la composition alimentaire selon l'invention possèdent une bonne valeur nutritionnelle même chez les sujets malades.

La quantité d'eau employée pour réaliser la composition est déterminée pour que la composition soit plus ou moins liquide et puisse être facilement ingérée par le patient.

Le pourcentage pondéral du mélange constitué de vitamines, de minéraux et d'électrolytes est choisi de façon à répondre aux proportions, connues de l'homme du métier, devant se trouver dans une alimentation équilibrée.

Préférentiellement, ladite composition contient moins de 100 picomoles/g de putrescine, moins de 100 picomoles/g de spermidine, moins de 100 picomoles/g de spermine, moins de 100 picomoles/g de cadavérine.

Une telle composition pourra être administrée conjointement à au moins un inhibiteur de la synthèse intracellulaire des polyamines.

Selon une variante intéressante de l'invention ladite composition est enrichie avec au moins un inhibiteur de la synthèse intracellulaire des polyamines à raison d'au plus 15% en poids par rapport au poids sec total de la composition et, préférentiellement, à raison d'une quantité comprise entre 0,2% et 7% en poids.

Les inhibiteurs de l'ODC utilisables sont choisis en particulier parmi les composés suivants :

### Antagonistes du phosphate de pyridoxal

. L-canaline
. N-(5'-phosphopyridoxyl)ornithine

### Inhibiteurs compétitifs

. α-hydrazino-ornithine
. acide DL-α-hydrazino-δ-aminovalérique
. α-méthylornithine (α-MO)
. *trans*-3-déhydro-DL-ornithine
. 1,4-diamino-*tran*s-2-butène
. 1,4-diaminobutanone

### Inhibiteurs diaminés

. 1,3-diaminopropane
. 1,3-diamino-2-propanol
. bis(éthyl)spermine

### Inhibiteurs suicides et irréversibles

. 2-difluorométhylornithine (DFMO)
. monofluorométhylornithine
. 2-monofluorométhyldéhydro-ornithine
. 2-monofluorométhyldéhydro-ornithine méthyl ester
. 5-hexyne-1,4-diamine
. *trans*-hex-2-èn-5-yne-1,4-diamine
. monofluorométhylputrescine
. difluorométhylputrescine
. α-allénylputrescine
. (2R,5R)-6-heptyne-2,5-diamine.

Parmi ces inhibiteurs, les inhibiteurs compétitifs sont particulièrement préférés et notamment l'α-méthylornithine (α-MO).

L'α-méthylornithine montre de nombreux avantages dans le cadre de l'utilisation proposée ici. En effet, l'α-MO présente l'intérêt d'être un composé naturel facilement synthétisable et a une constante d'inhibition élevée.

L'α-méthylornithine présente de plus l'avantage d'inhiber la synthèse des polyamines chez Escherischia coli, la bactérie la plus commune peuplant naturellement le tractus intestinal, ce qui n'est notamment pas le cas de l'α-DFMO.

Ainsi l'emploi d'une composition alimentaire selon l'invention contenant en tant qu'inhibiteur de la synthèse intracellulaire des polyamines, de l'α-méthylornithine est-elle susceptible de réduire l'apport exogène de polyamines par les bactéries intestinales sans pour autant avoir recours à une antibiothérapie concomitamment à l'administration de cette composition ou, pour le moins, en permettant de diminuer la dose d'antibiotiques administrée.

Enfin, l'α-MO présente l'avantage d'être un simple inhibiteur compétitif de l'ornithine décarboxylase (ce qui va à l'encontre des pistes actuellement explorées par la communauté scientifique internationale) et diminue fortement les risques d'accoutumance de l'organisme par mutation aboutissant à une résistance cellulaire accrue.

La conception originale de l'invention privilégie la recherche d'un effet de synergie entre plusieurs facteurs, à savoir notamment l'inhibition de la synthèse intracellulaire des polyamines et l'apport carencé conjoint en ces composés. Sous cet angle, il apparaît également que le meilleur inhibiteur est l'α-MO.

Selon une variante, la composition selon l'invention est enrichie en vitamines, notamment celles apportées, chez l'être humain sain, par les bactéries intestinales. En effet, l'antibiothérapie qui peut accompagner l'administration de ladite composition peut aussi conduire à diminuer l'apport en certaines vitamines. Dans ce cas, il peut s'avérer nécessaire d'enrichir la composition selon l'invention en ces vitamines afin de ne pas provoquer de carence vitaminique à la suite de l'administration prolongée de ladite composition. Notamment, il pourra s'avérer utile d'enrichir la composition en vitamines ou en dérivés de vitamines. Certains dérivés de la vitamine A (acide rétinoïque) sont en effet des inhibiteurs de l'activité ODC.

Préférentiellement, les glucides de la composition appartiennent au groupe comprenant les polymères de glucose, les maltodextrines, le saccharose, les amidons modifiés, le glucose monohydraté, le sirop de glucose déshydraté, le monostéarate de glycérol et leurs mélanges. De tels glucides sont en effet digestibles même en cas de pathologie digestive ou d'anomalies fonctionnelles secondaires à un traitement anticancéreux (chimiothérapie ou radiothérapie).

Selon une variante de l'invention, les protéines utilisées appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

Préférentiellement, les lipides appartiennent au groupe comprenant l'huile de beurre, l'huile d'arachide, les triglycérides à chaîne moyenne, l'huile de pépins de raisin, l'huile de soja, l'huile d'onagre et leurs mélanges. Avantageusement, lesdits lipides sont constitués par un mélange d'au moins une huile d'origine animale, d'au moins une huile d'origine végétale et de stéarate de glycérol.

Selon une variante de l'invention ladite composition constitue une ration journalière alimentaire d'un être humain et comprend :
- éventuellement ledit inhibiteur de la synthèse intracellulaire des polyamines à raison de moins de 50g et préférentiellement à raison de 1 à 10 g,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

Les quantités de vitamines, de minéraux et d'électrolytes utilisés sont connues de l'homme du métier et peuvent être facilement trouvées dans la littérature (voir par exemple,"Apports nutritionnels conseillés" Dupin, Abraham et Giachetti deuxième édition 1992, Ed. TEC et DOC Lavoisier)

Une telle composition permet, à elle seule, de répondre aux besoins nutritionnels journaliers d'un patient tout en permettant de réduire la synthèse intracellulaire et l'apport extérieur en polyamines. Elle constitue alors un aliment à part entière.

Bien sûr, il pourra être envisagé d'administrer une telle composition non pas en une seule prise mais en plusieurs prises espacées au cours de la même journée. Chaque ration sera alors définie pondéralement de façon à constituer un sous-multiple d'une ration journalière alimentaire d'un être humain et comprendra:
- éventuellement ledit inhibiteur de la synthèse intracellulaire des polyamines à raison de moins de 50/X g et préférentiellement à raison de 1/X à 10/X g,
- entre 75X g et 500/X g de glucides,
- entre 20/X g et 185/X g de lipides,
- entre 20X g et 225/X g de protéines,
- des vitamines, des minéraux et des électrolytes en quantités suffisantes pour répondre partiellement aux besoins nutritionnels journaliers d'un être humain. X étant un entier compris entre 2 et 8 et correspondant au nombre de rations devant être ingérées par le patient pour satisfaire ses besoins nutritionnels journaliers.

Le nombre de telles rations pourra être choisi de façon à répondre totalement aux besoins alimentaires journaliers du patient ou bien être choisi de façon à ne couvrir qu'une partie des besoins nutritionnels de celui-ci, le reste de ces besoins étant assurés par un alimentation naturelle pauvre en polyamines (jambon et pâtes ou riz par exemple). Dans ce cas la composition alimentaire sera utilisée en tant que complément alimentaire.

L'invention a également pour objet un agent à deux composants A et B, le composant A étant constitué par une composition pouvant être ingérée par l'homme constituée d'un mélange nutritif pauvre en polyamines contenant moins de 400 picomoles/g, préférentiellement moins de 100 picomoles/g de putrescine, moins de 400 picomoles/g, préférentiellement moins de 100 picomoles/g de spermidine, moins de 400 picomoles/g, préférentiellement moins de 100 picomoles/g de spermine, et moins de 400 picomoles/g, préférentiellement moins de 100 picomoles/g de cadavérine, et comprenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes, et un composé B, constitué par un inhibiteur de synthèse intracellulaire des polyamines, constitué de préférence par l'α-méthylornithine, les composants A et B étant mis en oeuvre comme produits de combinaison pour une utilisation simultanée, séparée ou décalée dans le temps pour le traitement du cancer, en particulier du cancer de la prostate.

La composition ou l'agent selon l'invention pourront être utilisés à titre de médicament, d'aliment, de supplément alimentaire ou encore de produit de nutrition thérapeutique.

La composition ou l'agent selon l'invention sont susceptibles de présenter une activité anti-néoplasique chez l'homme. La déplétion en polyamines potentialisant les effets des traitements anticancéreux conventionnels, la composition ou l'agent selon l'invention seront utilisables en association avec d'autres traitements anticancéreux, y compris ceux faisant appel à des inhibiteurs du métabolisme des polyamines.

En particulier, une telle composition ou un tel agent sont susceptibles de se révéler efficaces pour le traitement du cancer de la prostate.

De récents travaux ont par ailleurs permis de mettre en évidence qu'une telle composition utilisée dans le cadre d'un traitement visant à réduire toutes les voies d'apport exogène et endogène en polyamines était susceptible de présenter, chez l'homme, un effet thérapeutique constitué par la stimulation du système immunitaire. Notamment, ces travaux ont prouvé qu'une composition analogue administrée à des murins, concomitamment à l'administration d'un inhibiteur de la synthèse des polyamines (le DFMO) et à un antibiotique (la Néomycine) permettait de stimuler l'activité cytotoxique des cellules "natural killers" (cellules NK) et permettait également de stimuler et même de normaliser la production endogène d'interleukine 2.

D'autres travaux ont également permis de mettre en évidence qu'une telle composition, en particulier lorsqu'elle est administrée concomitamment à un inhibiteur de la synthèse des polyamines, induisait chez l'homme un effet antalgique puissant.

Par ailleurs, d'autres travaux ont également permis de mettre en évidence qu'une telle composition utilisée en complément avec un inhibiteur de la synthèse des polyamines était susceptible, chez l'homme, de réduire l'appétit.

L'invention sera plus facilement comprise grâce aux deux exemples de réalisation de l'invention qui vont suivre.

### Exemple 1

On prépare la composition suivante (voir tableau IV).
On disperse dans 150 litres d'eau,
- 786 g de phosphate disodique,
- 5,080 kg d'huile d'arachide,
- 0,300 kg de stéarate de glycérol,
- 1,280 kg d'huile de beurre, après agitation, on y incorpore :
- 8,760 kg de concentré de protéine soluble de lait, et ensuite 18,750 kg de maltodextrine, 7,000 kg de saccharose.

L'ensemble est refroidi à température ambiante et on ajoute 45 g d'oxyde de magnésium, 13,8 g de carbonate de calcium, 227,5 g de chlorure de magnésium, 6,7 g de sulfate de fer, 40 g de prémix vitaminique et de sels minéraux, 73,6 g de chlorure de choline, 60,0 g d'acide ascorbique.

On ajuste ensuite le volume de la cuve à 200 litres, par addition d'eau, et le pH est ajusté à 7,10 par addition d'une solution d'hydroxyde de sodium 2N.

Après dégazage, stérilisation, homogénéisation, on introduit le produit dans des boîtes métalliques serties. On ajoute à cette composition, lorsqu'on souhaite une composition complète, 4,0kg d'α-méthylornithine.

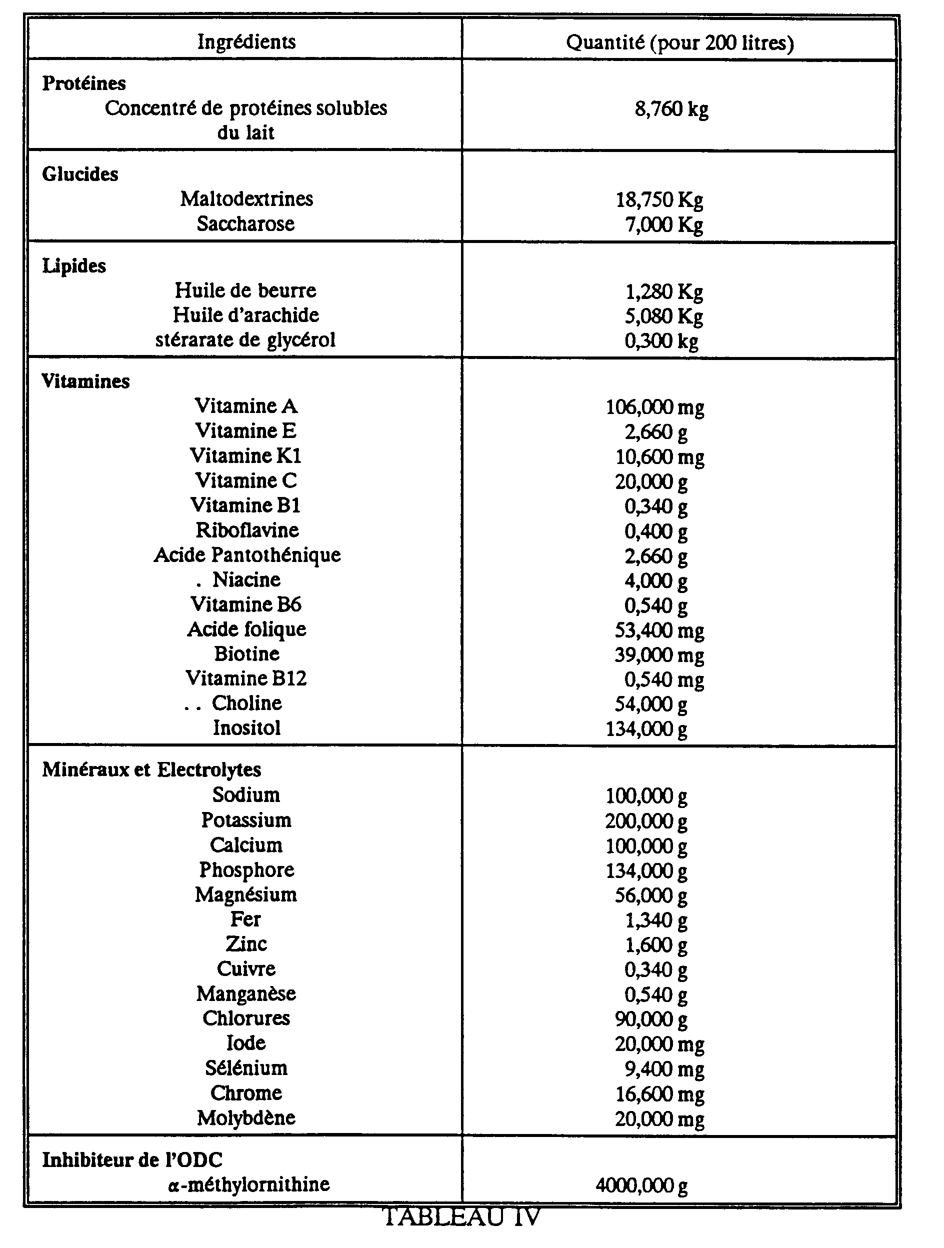

La composition centésimale obtenue est la suivante :
Glucides : 12,500 g dont 9,000 g de maltodextrines et 3,500 g de saccharose ;
Lipides : 3,330 g dont 0,640 g d'huile de beurre, 2,540 g d'huile d'arachide et 0,150 g de stéarate de glycérol ;
Protéines : 4,000 g apportées par un concentré de protéines solubles de lait.
Vitamines, minéraux et électrolytes : 0,600 g ;
α-méthylornithine : 3,000 g ;
eau : QSP 100 ml.

Le pourcentage pondéral de vitamines, de minéraux et d'électrolytes utilisé est choisi de façon telle que la consommation journalière de quatre boîtes permette de répondre aux besoins nutritionnels conseillés par Dupin, Abraham et Giachetti ("Apports nutritionnels conseillés pour la population française" deuxième édition 1992, Editions TEC et DOC Lavoisier).

### Exemple 2

Une préparation présentant sensiblement les mêmes ingrédients que ceux indiqués dans le tableau IV est préparée selon la même procédé que celui détaillé dans l'exemple 1. Toutefois, la source de protéines est remplacée par un mélange d'isolat de soja et de poudre de jaune d'oeufs. L'huile d'arachide est quant à elle substituée par un mélange d'huile de germes de maïs et d'huile de soja.

La composition centésimale obtenue par boîte de 100 ml est la suivante :
Glucides : 12,500 g dont 9,000 g de maltodextrines et 3,500 g de saccharose ;
Lipides : 3,330 g dont 0,640 g d'huile de beurre, 0,840 g d'huile de germes de maïs, 1,700 g d'huile de soja et 0,150 g ;
Protéines : 4,000 g dont 3,380 g d'isolat de soja et 0,620 g de poudre de jaune d'oeufs ;
Vitamines, minéraux et électrolytes : 0,600g
α-méthylornithine : 3,000 g
eau : QSP 100 ml.

De nombreux tests ont été menés chez le rat et la souris à l'aide de compositions analogues à celles décrites aux exemples 1 et 2 adaptées aux besoins nutritionnels des murins.

Ces différents tests ont permis de mettre en évidence différents effets thérapeutiques induits par un traitement visant à carencer l'organisme en polyamines.

### Effet sur l'inhibition de la croissance tumorale.

Des souris porteuses du carcinome pulmonaire de Lewis 3LL sont traitées par une composition alimentaire de synthèse pauvre en polyamines (AP), conforme à la présente invention dont la formule répond aux besoins nutritionnels humains, de l'α MO à 0,5% et des antibiotiques (ATB) (Néomycine : 0,2%, Flagyl 0,0034%). Les témoins reçoivent un aliment identique à celui des traités mais dans lequel des quantités de polyamines 200 à 1800 fois supérieurs ont été rajoutées afin d'aboutir à une consommation en polyamines équivalente à celle obtenue en cas d'alimentation standard. Avec l'aliment déplété, la consommation journalière en putrescine est équivalente à celle qui serait obtenue chez l'homme avec un aliment de synthèse contenant 50 picomoles de putrescine par gramme.

Après 15 jours de traitement, les animaux ont été sacrifiés et les tailles de leurs tumeurs ont été mesurées. Les résultats sont donnés dans le tableau V qui montre une diminution très importante de la taille des tumeurs dans le cas où l'alimentation a été administrée avec des antibiotiques et/ou de l'α MO.

**TABLEAU V**

| | Aliment Pauvre | | Aliment Pauvre + ATB | |
|---|---|---|---|---|
| | volume tumoral | % d'inhibition | Volume tumoral | % d'inhibition |
| Témoins (AP + polyamines) | 2.13 ± 0,66 | 0 | | |
| AP | 2.10 ± 0,46 | 0 | 0.82 ± 0,46** | 61% |
| AP + α MO | 0.63 ± 0,2** | 70% | 0.49 ± 0,33 | 77% |

| | | | | |
|---|---|---|---|---|
| ** différence significative (p < 0,05) | | | | |

### Effet sur la stimulation de l'activité des cellules NK

Des souris porteuses du carcinome pulmonaire de Lewis 3LL sont traitées par un aliment déplété en polyamines (*), du DFMO à 3% dans l'eau de boisson et des antibiotiques (Néomycine:0,2%, Flagyl 0,0034% dans l'eau de boisson). Cet aliment contient 30 à 50 fois moins de polyamines que l'aliment standard reçu par les témoins : la consommation journalière en putrescine est équivalente à celle qui serait obtenue chez l'homme avec un aliment de synthèse contenant 380 pmoles de putrescine par g. L'aliment standard ainsi que l'aliment déplété répondent aux besoins nutritionnels murins.

Les résultats (moyenne ± déviation standard) sont obtenus sur n=4 animaux par lot expérimental.

Les résultats (volume tumoral, taux tissulaires de putrescine et activité des cellules NK) après 15 jours de traitement sont donnés dans le tableau VI. Ce tableau montre une forte stimulation de l'activité des cellules NK par le traitement et l'administration conjointe d'antibiotiques (Néomycine, Flagyl) et de DFMO à 3 %.

**TABLEAU VI**

| | Volume tumoral (cm^{*3*}) | Taux tissulaires de putrescine | | Rein | Activité NK (% de lyse) |
|---|---|---|---|---|---|
| | | Tumeur | Foie (nmoles/g) | | |
| Témoins (Aliment standard) | 5,2 ± 3,1 | 25,4 ± 20 | 24,9 ± 11 | 14,2 ± 1,5 | 2,6 ± 2,3 |
| Aliment déplété* + DFMO3% + ATB | 0,4 ± 0,7** | 11,6 ± 5 | 5,9 ± 0,2** | 5,9 ± 1,4** | 13,4 ±2,1** |

| | | | | | |
|---|---|---|---|---|---|
| ** différence significative (p < 0,05) | | | | | |

Des souris porteuses du carcinome pulmonaire de Lewis 3LL sont traitées pendant 7 jours par un aliment déplété en polyamines (^{*}), du DFMO à 3% dans l'eau de boisson et des antibiotiques (néomycine : 0,2%, Flagyl 0,0034% dans l'eau de boisson). Cet aliment contient 20 à 100 fois moins de polyamines que l'aliment standard reçu par les témoins : la consommation journalière en putrescine est équivalente à celle qui serait obtenue chez l'homme avec un aliment de synthèse contenant 175 pmoles de putrescine par g.

Les rats porteurs du carcinome prostatique Mat Lylu sont traités pendant 14 jours par un aliment déplété en polyamines (^{**}), du DFMO à 3% dans l'eau de boisson et des antibiotiques (Néomycine : 0,2%; Flagyl 0,0034% dans l'eau de boisson). cet aliment contient 30 à 50 fois moins de polyamines que l'aliment standard reçu par les témoins : la consommation journalière en putrescine est équivalente à celle qui serait obtenue chez l'homme avec un aliment de synthèse contenant 380 pmoles de putrescine par g.

L'aliment standard ainsi que l'aliment déplété répondent aux besoins nutritionnels murins.

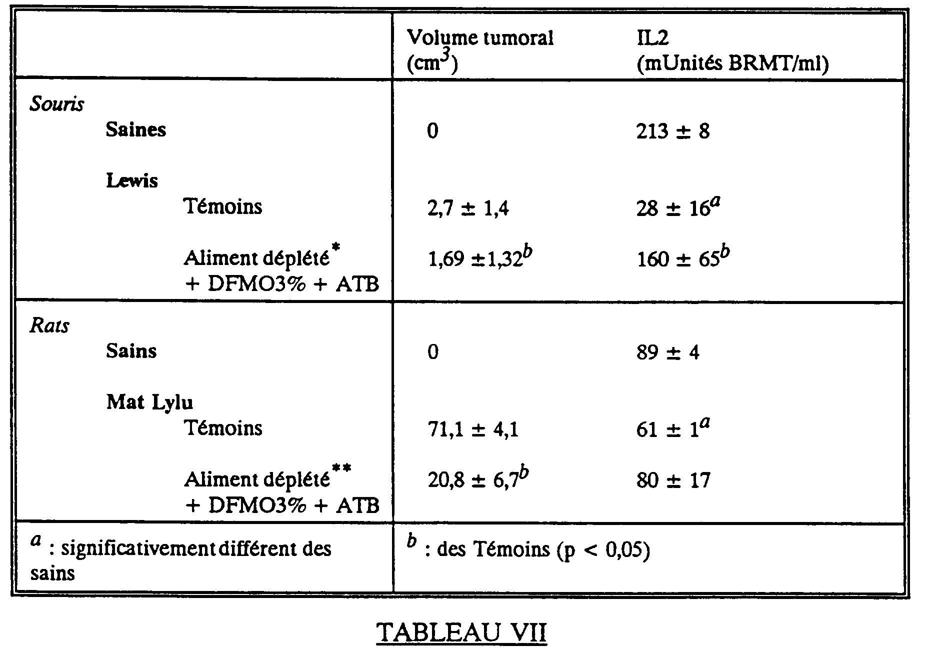

Les résultats sont donnés dans le Tableau VII. Ce tableau montre une très nette stimulation de la production endogène d'interleukine 2 chez les souris porteuses du carcinome pulmonaire de Lewis 3LL et une quasi normalisation de la production endogène d'interleukine 2 chez les rats porteurs du carcinome prostatique Mat Lylu, lorsque les animaux sont traités avec l'aliment déplété en polyamines avec administration conjointe d'antibiotiques (Néomycine et Flagyl) et de DFMO 3%.

### Effet antalgique

Le test du "tail flick" (sensibilité à un stimulus algique produit par un faisceau lumineux) a été effectué à l'intensité 7 chez des rats mâles de souche Wistar pesant en moyenne 300 g, nourris *per os ad libitum* soit avec (1) un aliment solide standard, soit avec (2) un aliment solide contenant 30 à 50 fois moins de polyamines que l'aliment standard, soit avec (3) un aliment liquide contenant 200 à 1800 fois moins de polyamines que l'aliment solide standard. Les aliments (1) et (2) répondent aux besoins nutritionnels journaliers des murins, l'aliment (3) aux besoins nutritionnels journaliers humains(4). Qu'il s'agisse d'alimentation solide ou liquide, les animaux reçoivent journellement *per os* la même quantité d'α-DFMO (2 g.Kg⁻¹.5⁻¹) contenue soit dans l'eau de boisson (en cas d'alimentation solide), soit dans l'aliment liquide lui-même.

Les résultats après 7 jours de traitement traduisant l'évolution de la résistance à la douleur sont donnés dans le Tableau VIII.

**TABLEAU VIII**

| Traitement | Test du tail-flick (secondes) |
|---|---|
| Aliment solide standard (^{*1*}) | 11,25 ± 2,32 |
| Aliment solide pauvre en polyamines (^{*2*}) | 11,80 ± 1,92 |
| Aliment solide pauvre en polyamines (^{*2*}) + α-DFMO (^{*4*}) | 11,57 ± 2,31 |
| Aliment liquide pauvre en polyamines (^{*3*}) | 18,08 ± 2,69 * |
| Aliment liquide pauvre en polyamines (^{*3*}) + α-DFMO (^{*4*}) | 18,58 ± 2,17 * |

| | |
|---|---|
| (^{*}) p < 5% par rapport aux groupes recevant une alimentation solide. | |

Ce tableau montre une nette augmentation du seuil de résistance à la douleur chez les animaux nourris avec un aliment pauvre en polyamines, surtout lorsque celui-ci est accompagné de l'administration d'α-DFMO.

Le test de résistance à la pression (application d'une masselotte de 140 g sur les coussinets plantaires des animaux) a également été réalisé chez des rats mâles de souche Wistar pesant en moyenne 300 g, nourris *per os ad libitum* soit avec (1) un aliment solide standard, soit avec (2) un aliment solide contenant 30 à 50 fois moins de polyamines que l'aliment standard, soit avec (3) un aliment liquide contenant 200 à 1800 fois moins de polyamines que l'aliment solide standard. Les aliments (1) et (2) répondent aux besoins nutritionnels journaliers des murins, l'aliment (3) aux besoins nutritionnels journaliers humains. (4) Qu'il s'agisse d'alimentation solide ou liquide, les animaux reçoivent journellement *per os* la même quantité d'α-DFMO (2g.Kg⁻¹.j⁻¹l) contenue soit dans l'eau de boisson (en cas d'alimentation solide), soit dans l'aliment liquide lui-même.

Les résultats après 7 jours de traitement traduisant l'évolution de la résistance à la douleur sont donnés dans le tableau IX.

**TABLEAU IX**

| Traitement | Résistance à la pression (Newtons) |
|---|---|
| Aliment solide standard (^{*1*}) | 0,12 ± 0,01 |
| Aliment solide pauvre en polyamines (^{*2*}) | 0,10 ± 0,01 * |
| Aliment solide pauvre en polyamines (^{*2*}) + α-DFMO (^{*4*}) | 0,33 ± 0,08 * |
| Aliment liquide pauvre en polyamines (^{*3*}) | 0.21 ± 0,01 * |
| Aliment liquide pauvre en polyamines (^{*3*}) + α-DFMO (^{*4*}) | 0,24 ± 0,01 * |

| | |
|---|---|
| (^{*}) p < 5% par rapport aux groupes recevant une alimentation solide | |

Ce tableau montre également une augmentation sensible de la résistance à la douleur chez les animaux nourris avec une alimentation pauvre en polyamines, particulièrment lorsque celle-ci est associée à l'administration de DFMO.

### Effet sur l'évolution du poids corporel et la consommation alimentaire

Cinq lots de rats ont été alimentés avec une composition alimentaire pauvre en polyamines et traités avec du DFMO pendant 20 jours. Leur consommation alimentaire et l'évolution de leur poids ont été comparés avec ceux de cinq lots de rats ayant reçu la même alimentation mais dans laquelle des quantités de polyamines ont été rajoutées afin d'aboutir à une consommation en polyamines équivalente à celle obtenue en cas d'alimentation standard.

La consommation alimentaire journalière moyenne des lots traités avec la composition pauvre en polyamine et le DFMO a été de 19,48 ± 5,70 g d'aliment et de 20,52 ± 9,43 ml de boisson contre respectivement 28,53 ± 3,46 g d'aliments et de 32,72 ± 5,51 ml de boisson chez les lots non-traités.

Par ailleurs, les rats traités ont gardé un poids d'environ 350 g en moyenne tout au long du traitement alors que les rats non traités ont vu leur poids passer en moyenne d'environ 350 g à plus de 400 g.

Les deux exemples de réalisation de la composition décrits ci-dessus n'ont pas pour objet de réduire la portée de l'invention. En particulier, il pourra être envisagé d'employer un autre inhibiteur de la synthèse intracellulaire des polyamines que l'α-méthylornithine ou l'α-DFMO et d'incorporer cet inhibiteur dans des proportions pondérales plus importantes que celle mentionnée dans ces exemples. Il pourra également être envisagé d'utiliser d'autres sources de glucides, de lipides ou de protéines que celles mentionnées sans sortir du cadre de l'invention.

## Revendications

1. Composition pouvant être ingérée par l'homme caractérisée en ce quelle est constituée d'un mélange nutritif pauvre en polyamines contenant moins de 1600 picomoles/g de polyamines.

2. Composition selon la revendication 1 caractérisée en ce qu'elle contient moins de 400 picomoles/g de putrescine, moins de 400 picomoles/g de spermidine, moins de 400 picomoles/g de spermine et moins de 400 picomoles/g de cadavérine.

3. Composition selon la revendication 1 ou 2 caractérisée en ce qu'elle contient moins de 400, préférentiellement moins de 200, picomoles/g de polyamines.

4. Composition selon la revendication 3 caractérisée en ce qu'elle contient moins de 100, préférentiellement moins de 50, picomoles/g de putrescine, moins de 100, préférentiellement moins de 50, picomoles/g de spermidine, moins de 100, préférentiellement moins de 50, picomoles/g de spermine et moins de 100, préférentiellement moins de 50, picomoles/g de cadavérine.

5. Composition selon l'une des revendications 1 à 4 caractérisée en ce qu'elle contient, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

6. Composition selon l'une des revendications 1 à 5 caractérisée en ce qu'elle est enrichie avec au moins un inhibiteur de la synthèse intracellulaire des polyamines à raison d'au plus 15 % en poids par rapport au poids sec total de la composition.

7. Composition selon la revendication 6 caractérisée en ce qu'elle est enrichie avec ledit inhibiteur à raison de 0,2 % à 7 % en poids par rapport au poids sec total de la composition.

8. Composition selon la revendication 6 ou 7 caractérisée en ce que ledit inhibiteur est un inhibiteur compétitif de l'ornithine décarboxylase.

9. Composition selon la revendication 8 caractérisée en ce que ledit inhibiteur compétitif est l'α-méthylornithine.

10. Composition selon l'une des revendications 1 à 9 caractérisée en ce qu'elle contient au moins un antibiotique.

11. Composition selon l'une des revendications 1 à 10 caractérisée en ce qu'elle est enrichie en vitamines.

12. Composition selon l'une des revendications 1 à 11 caractérisée en ce que lesdits glucides appartiennent au groupe comprenant les polymères de glucose, les maltodextrines, le saccharose, les amidons modifiés, le glucose monohydraté, le sirop de glucose déshydraté, le monostéarate de glycérol et leurs mélanges.

13. Composition selon l'une des revendications 1 à 12 caractérisée en ce que lesdites protéines appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

14. Composition selon l'une quelconque des revendications 1 à 13 caractérisée en ce que lesdits lipides appartiennent au groupe comprenant l'huile de beurre, l'huile d'arachide, les triglycérides à chaîne moyenne, l'huile de pépins de raisin, l'huile de soja, l'huile d'onagre et leurs mélanges.

15. Composition selon l'une des revendications 1 à 14 caractérisée en ce que lesdits lipides sont constitués par un mélange d'au moins une huile d'origine animale, d'au moins une huile d'origine végétale et de stéarate de glycérol.

16. Composition selon l'une des revendications 1 et 10 à 15 caractérisée en ce qu'elle constitue la ration journalière alimentaire d'un être humain et en ce qu'elle comprend :
- entre 75 g et 500 g de glucides
- entre 20 g et 185 g de lipides
- entre 20 g et 225 g de protéines
- des vitamines, des minéraux et des électrolytes en quantités suffisantes pour répondre aux besoins nutritionnels journaliers d'un être humain.

17. Composition selon l'une des revendications 1 à 16 caractérisée en ce qu'elle constitue la ration journalière alimentaire d'un être humain et en ce qu'elle comprend :
- moins de 50 g et, préférentiellement, entre 1 à 10 g dudit inhibiteur de la synthèse intracellulaire des polyamines,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

18. Composition selon l'une des revendications 1 et 10 à 15 caractérisée en ce qu'elle est un sous-multiple d'une ration journalière alimentaire d'un être humain et en ce qu'elle comprend :
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain, et X étant un entier compris entre 2 et 8 et correspondant au nombre de rations devant être ingérées par le patient pour satisfaire ses besoins nutritionnels journaliers.

19. Composition selon l'une des revendications 1 à 18 caractérisée en ce qu'elle est un sous-multiple d'une ration journalière alimentaire d'un être humain et en ce qu'elle comprend :
- moins de 50/X g et, préférentiellement, entre 1/X et 10/X g dudit inhibiteur de la synthèse intracellulaire des polyamines,
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain, et X étant un entier compris entre 2 et 8 et correspondant au nombre de rations devant être ingérées par le patient pour satisfaire ses besoins nutritionnels journaliers.

20. Composition selon l'une des revendications 1 à 19 caractérisée en ce qu'elle se présente sous une forme sèche à dissoudre extemporanément dans un véhicule neutre.

21. Composition selon l'une des revendications 1 à 19 caractérisée en ce qu'elle inclut un véhicule neutre la rendant prête à l'emploi.

22. Agent à deux composants A et B, le composant A étant constitué par une composition pouvant être ingérée par l'homme selon l'une des revendications 1, 10 à 16, 18, 20 ou 21 et le composé B étant constitué par un inhibiteur de synthèse intracellulaire des polyamines, les composants A et B étant mis en oeuvre comme produits de combinaison pour une utilisation simultanée, séparée ou décalée dans le temps.

23. Agent selon la revendication 22 caractérisé en ce que ledit inhibiteur est de l'-αméthylornithine.

24. Composition ou agent selon l'une quelconque des revendications 1 à 23 pour une utilisation appartenant au groupe comprenant les aliments, suppléments alimentaires et produits de nutrition.

25. Composition ou agent selon l'une quelconque des revendications 1 à 23 utilisable comme médicament.

26. Composition ou agent selon l'une des revendications 24 ou 25 utilisable comme produit de nutrition thérapeutique.

27. Composition ou agent selon l'une quelconque des revendications 24 à 26 utilisable comme agent anti-cancéreux.

28. Composition ou agent selon la revendication 27 utilisable pour le traitement du cancer de la prostate.

29. Composition ou agent selon l'une des revendications 24 à 27 utilisable comme agent visant à stimuler le système immunitaire.

30. Composition ou agent selon la revendication 29 utilisable comme agent visant à stimuler l'activité des cellules NK.

31. Composition ou agent selon la revendication 29 ou 30 utilisable comme agent visant à stimuler la production endogène d'interleukine 2.

32. Composition ou agent selon l'une des revendications 24 à 27 utilisable comme agent antalgique.

33. Composition ou agent selon l'une des revendications 24 à 27 utilisable comme agent visant à réduire l'appétit.

## Claims

1. Composition that can be ingested by man, characterized in that it consists of a nutritive mixture low in polyamines and containing less than 1600 picomoles/g of polyamines.

2. Composition according to claim 1, characterized in that it contains less than 400 picomoles/g of putrescine, less than 400 picomoles/g of spermidine, less than 400 picomoles/g of spermine, and less than 400 picomoles/g of cadaverine.

3. Composition according to claim 1 or 2, characterized in that it contains less than 400, and preferably less than 200, picomoles/g of polyamines.

4. Composition according to claim 3, characterized in that it contains less than 100 and preferably less than 50 picomoles/g of putrescine; less than 100 and preferably less than 50 picomoles/g of spermidine; less than 100 and preferably less than 50 picomoles/g of spermine; and less than 100 and preferably less than 50 picomoles/g of cadaverine.

5. Composition according to one of claims 1 to 4, characterized in that it contains, as a percentage dry weight based on the total dry weight: 10% to 35% of lipids, 8% to 30% of proteins, 35% to 80% of glucides, and up to 10% of a mixture consisting of vitamins, minerals and electrolytes.

6. Composition according to one of claims 1 to 5 characterized in that it is enriched with not more than 15% by weight, based on the total dry weight of the composition, of at least one intracellular polyamine synthesis inhibitor.

7. Composition according to claim 6, characterized in that it is enriched with 0.2% to 7% by weight of the said inhibitor based on the total dry weight of the composition.

8. Composition according to claim 6 or 7, characterized in that the said inhibitor is a competitive inhibitor of ornithine-decarboxylase.

9. Composition according to claim 8, characterized in that the said competitive inhibitor is α-methylornithine.

10. Composition according to one of claims 1 to 9, characterized in that it contains at least one antibiotic.

11. Composition according to one of claims 1 to 10, characterized in that it is enriched in vitamins.

12. Composition according to one of claims 1 to 11, characterized in that the said glucides belong to the group comprising glucose polymers, maltodextrins, sucrose, modified starches, monohydrated glucose, dehydrated glucose syrup, glycerol monostearate and mixtures thereof.

13. Composition according to one of claims 1 to 12, characterized in that the said proteins belong to the group comprising milk soluble proteins, soya proteins, serum peptides, powdered egg yolk, potassium caseinate, non-phosphorylated peptides, casein peptides, mixed caseinate, soya isolate and mixtures thereof.

14. Composition according to any one of claims 1 to 13, characterized in that the said lipids belong to the group comprising butter oil, peanut oil, medium chain triglycerides, grapeseed oil, soya oil, evening primrose oil and mixtures thereof.

15. Composition according to one of claims 1 to 14, characterized in that the said lipids consist of a mixture of at least one oil of animal origin, at least one oil of vegetable origin and glycerol stearate.

16. Composition according to one of claims 1 and 10 to 15, characterized in that it constitutes the daily food ration of a human being and in that it comprises:
- between 75 g and 500 g of glucides,
- between 20 g and 185 g of lipids,
- between 20 g and 225 g of proteins,
- vitamins, minerals and electrolytes in sufficient quantities to satisfy the daily nutritional needs of a human being.

17. Composition according to one of claims 1 to 16, characterized in that it constitutes the daily food ration of a human being and in that it comprises :
- less than 50 g, and preferably between 1 and 10 g, of the said intracellular polyamine synthesis inhibitor, between 75 g and 500 g of glucides,
- between 20 and 185 g of lipids,
- between 20 and 225 g of proteins,
- vitamins, minerals and electrolytes in sufficient quantities to satisfy the daily nutritional needs of a human being.

18. Composition according to one of claims 1 and 10 to 15, characterized in that it is a submultiple of a daily food ration of a human being and in that it comprises :
- between 75/X g and 500/X g of glucides,
- between 20/X g and 185/X g of lipids
- between 20/X g and 225/X g of proteins,
- vitamins, minerals and electrolytes in sufficient quantities to satisfy partially the daily nutritional needs of a human being, X being an integer between 2 and 8 corresponding to the number of rations which should be ingested by the patient to satisfy his daily nutritional needs.

19. Composition according to one of claims 1 to 18, characterized in that it is a submultiple of a daily food ration for a human being and in that it comprises :
- less than 50/X g and preferably between 1/X g and 10/X g of the said intracellular polyamine synthesis inhibitor,
- between 75/X g and 500/X g of glucides,
- between 20/X g and 185/X g of lipids,
- between 20/X g and 225/X g of proteins,
- vitamins, minerals and electrolytes in sufficient quantities to satisfy partially the daily nutritional needs of a human being, X being an integer between 2 and 8 corresponding to the number of rations which should be ingested by the patient to satisfy his daily nutritional needs.

20. Composition according to one of claims 1 to 19, characterized in that it is in a dry form to be dissolved extemporaneously in a neutral vehicle.

21. Composition according to one of claims 1 to 19, characterized in that it includes a neutral vehicle making it ready for use.

22. Agent with two components A and B, component A consisting of a composition that can be ingested by man according to one of claims 1, 10 to 16, 18, 20 or 21, and compound B consisting of an intracellular polyamine synthesis inhibitor, components A and B being used as combined products for use simultaneously, separately or with a time lag.

23. Agent according to claim 22, characterized in that the said inhibitor is α-methylornithine.

24. Composition or agent according to any one of claims 1 to 23 for a use belonging to the group including foods, food supplements and nutritional products.

25. Composition or agent according to any one of claims 1 to 23 usable as a drug.

26. Composition or agent according to either of claims 24 or 25 usable as a therapeutic nutritional product.

27. Composition or agent according to any one of claims 24 to 26, usable as an anti-cancer agent.

28. Composition or agent according to claim 27 usable for the treatment of cancer of the prostate.

29. Composition or agent according to one of claims 24 to 27, usable as an agent designed to stimulate the immune system.

30. Composition or agent according to claim 29, usable as an agent designed to stimulate the activity of NK cells.

31. Composition or agent according to claim 29 or 30 usable as an agent designed to stimulate the endogenous production of interleukine 2.

32. Composition or agent according to one of claims 24 to 27 usable as an antalgic agent.

33. Composition or agent according to one of claims 24 to 27 usable as an agent designed to reduce appetite.

## Patentansprüche

1. Zusammensetzung, die vom Menschen eingenommen werden kann, dadurch gekennzeichnet, daß sie aus einem polyaminarmen Nährstoffgemisch besteht, das weniger als 1600 Pikomol/g an Polyaminen enthält.

2. Zusammensetzung gemäß Anspruch 1,
dadurch gekennzeichnet, daß sie weniger als 400 Pikomol/g Putrescin, weniger als 400 Pikomol/g Spermidin, weniger als 400 Pikomol/g Spermin und weniger als 400 Pikomol/g Kadaverin enthält.

3. Zusammensetzung gemäß Anspruch 1 oder 2,
dadurch gekennzeichnet, daß sie weniger als 400, bevorzugterweise weniger als 200 Pikomol/g an Polyaminen enthält.

4. Zusammensetzung gemäß Anspruch 3,
dadurch gekennzeichnet, daß sie weniger als 100, bevorzugterweise weniger als 50 Pikomol/g an Putrescin, weniger als 100, bevorzugterweise weniger als 50 Pikomol/g an Spermidin, weniger als 100, bevorzugterweise weniger als 50 Pikomol/g an Spermin und weniger als 100, bevorzugterweise weniger als 50 Pikomol/g an Kadaverin enthält.

5. Zusammensetzung gemäß einem der Ansprüche 1 bis 4,
dadurch gekennzeichnet, daß sie in Prozent des Trockengewichtes, im Verhältnis zum gesamten Trockengewicht, folgendes enthält: 10 % bis 35 % an Lipiden, 8 % bis 30 % an Proteinen, 35 % bis 80 % an Kohlehydraten und bis zu 10 % einer Mischung aus Vitaminen, Mineralstoffen und Elektrolyten.

6. Zusammensetzung gemäß einem der Ansprüche 1 bis 5,
dadurch gekennzeichnet, daß sie mit mindestens einem Hemmstoff der Polyaminsynthese innerhalb der Zellen angereichert ist, im Verhältnis von höchstens 15 % des Trockengewichtes der Zusammensetzung.

7. Zusammensetzung gemäß Anspruch 6,
dadurch gekennzeichnet, daß sie mit dem erwähnten Hemmstoff angereichert ist, im Verhältnis von 0,2 % bis 7 % des gesamten Trockengewichtes der Zusammensetzung.

8. Zusammensetzung gemäß Anspruch 6 oder 7,
dadurch gekennzeichnet, daß es sich bei dem erwähnten Hemmstoff um einen Hemmstoff handelt, der mit der Ornithin-Decarboxylase in Konkurrenz steht.

9. Zusammensetzung gemäß Anspruch 8,
dadurch gekennzeichnet, daß es sich bei dem konkurrierenden Hemmstoff um α-Methylornithin handelt.

10. Zusammensetzung gemäß einem der Ansprüche 1 bis 9,
dadurch gekennzeichnet, daß sie mindestens ein Antibiotikum enthält.

11. Zusammensetzung gemäß einem der Ansprüche 1 bis 10,
dadurch gekennzeichnet, daß sie mit Vitaminen angereichert ist.

12. Zusammensetzung gemäß einem der Ansprüche 1 bis 11,
dadurch gekennzeichnet, daß die erwähnten Kohlehydrate der Gruppe angehören, welche Glucosepolymere, Maltodextrine, Saccharosen, geänderte Stärken, Monohydratglucose, entwässerten Glucosesirup, Glycerinmonostearat und deren Mischungen enthält.

13. Zusammensetzung gemäß einem der Ansprüche 1 bis 12,
dadurch gekennzeichnet, daß die erwähnten Proteine der Gruppe angehören, welche lösliche Milchproteine, Sojaproteine, Serumpeptide, Eigelbpulver, Kaliumkaseinat, nicht phosphorylierte Peptide, Kaseinpeptide, gemischtes Kaseinat, Sojaisolat und deren Mischungen umfaßt.

14. Zusammensetzung gemäß einem der Ansprüche 1 bis 13,
dadurch gekennzeichnet, daß die erwähnten Lipide der Gruppe angehören, welche Butteröl, Erdnußöl, Triglyzeride mit mittleren Ketten, Traubenkernöl, Sojaöl, Oenotheraöl und deren Mischungen umfaßt.

15. Zusammensetzung gemäß einem der Ansprüche 1 bis 14,
dadurch gekennzeichnet, daß die erwähnten Lipide aus einer Mischung von mindestens einem tierischen Öl, mindestens einem pflanzlichen Öl und Glyzerinstearat bestehen.

16. Zusammensetzung gemäß einem der Ansprüche 1 und 10 bis 15,
dadurch gekennzeichnet, daß sie die tägliche Nahrungsration eines Menschen darstellt, wobei sie folgendes enthält:
- 75 g bis 500 g Kohlehydrate,
- 20 g bis 185 g Lipide,
- 20 g bis 225 g Proteine,
- Vitamine, Mineralstoffe und Elektrolyte in ausreichenden Mengen, um den täglichen Nährstoffbedarf eines Menschen zu befriedigen.

17. Zusammensetzung gemäß einem der Ansprüche 1 bis 16,
dadurch gekennzeichnet, daß sie die tägliche Nahrungsration eines Menschen darstellt, wobei sie folgendes enthält:
- weniger als 50 g, bevorzugterweise 1 bis 10 g des Hemmstoffes der intrazellulären Polyaminsynthese,
- 75 g bis 500 g Kohlehydrate,
- 20 g bis 185 g Lipide,
- 20 g bis 225 g Proteine,
- Vitamine, Mineralstoffe und Elektrolyte in ausreichenden Mengen, um den täglichen Nährstoffbedarf eines Menschen zu befriedigen.

18. Zusammensetzung gemäß einem der Ansprüche 1 und 10 bis 15,
dadurch gekennzeichnet, daß sie einen Bruchteil dertäglichen Nahrungsration eines Menschen darstellt, wobei sie folgendes enthält:
- 75/X g bis 500/X g Kohlehydrate,
- 20/X g bis 185/X g Lipide,
- 20/X g bis 225/X g Proteine,
- Vitamine, Mineralstoffe und Elektrolyte in ausreichenden Mengen, um den täglichen Nährstoffbedarf eines Menschen teilweise zu befriedigen,
wobei X eine zwischen 2 und 8 liegende ganze Zahl ist und der Zahl der Rationen entspricht, die vom Patienten einzunehmen sind, um seinen täglichen Nährstoffbedarf zu befriedigen.

19. Zusammensetzung gemäß einem der Ansprüche 1 bis 18,
dadurch gekennzeichnet, daß sie einen Bruchteil dertäglichen Nahrungsration eines Menschen darstellt, wobei sie folgendes enthält:
- weniger als 50/X g, bevorzugterweise 1/X bis 10/X g des Hemmstoffes der intrazellulären Polyaminsynthese,
- 75/X g bis 500/X g Kohlehydrate,
- 20/X g bis 185/X g Lipide,
- 20/X g bis 225/X g Proteine,
- Vitamine, Mineralstoffe und Elektrolyte in ausreichenden Mengen, um den täglichen Nährstoffbedarf eines Menschen teilweise zu befriedigen,
wobei X eine zwischen 2 und 8 liegende ganze Zahl ist und der Zahl der Rationen entspricht, die vom Patienten einzunehmen sind, um seinen täglichen Nährstoffbedarf zu befriedigen.

20. Zusammensetzung gemäß einem der Ansprüche 1 bis 19,
dadurch gekennzeichnet, daß sie in trockener Form vorliegt, die nach Bedarf in einem neutralen Trägerstoff gelöst wird.

21. Zusammensetzung gemäß einem der Ansprüche 1 bis 19,
dadurch gekennzeichnet, daß sie einen neutralen Trägerstoff umfaßt, welches sie gebrauchsfertig macht.

22. Mittel mit zwei Komponenten A und B, wobei die Komponente A aus einer Zusammensetzung besteht, die vom Menschen eingenommen werden kann, gemäß einem der Ansprüche 1, 10 bis 16, 18, 20 oder 21 und wobei die Komponente B aus einem Hemmstoff der intrazellulären Polyaminsynthese besteht, wobei die Komponenten A und B als Kombinationsprodukte zur gleichzeitigen, zeitlich getrennten oder verschobenen Anwendung eingesetzt werden.

23. Mittel gemäß Anspruch 22,
dadurch gekennzeichnet, daß es sich beim Hemmstoff um α-Methylornithin handelt.

24. Zusammensetzung oder Mittel gemäß einem der Ansprüche 1 bis 23, für eine Anwendung, die der Gruppe angehört, die Nährmittel, Nährmittelzusätze und Nährstoffe umfaßt.

25. Zusammensetzung oder Mittel gemäß einem der Ansprüche 1 bis 23, die als Medikament anwendbar ist.

26. Zusammensetzung oder Mittel gemäß einem der Ansprüche 24 oder 25, die als therapeutischer Nährstoff anwendbar ist.

27. Zusammensetzung oder Mittel gemäß einem der Ansprüche 24 bis 26, die als krebshemmendes Mittel anwendbar ist.

28. Zusammensetzung oder Mittel gemäß Anspruch 27, die zur Behandlung von Prostatakrebs anwendbar ist.

29. Zusammensetzung oder Mittel gemäß einem der Ansprüche 24 bis 27, die als Mittel zum Stimulieren des Immunsystems anwendbar ist.

30. Zusammensetzung oder Mittel gemäß Anspruch 29, die als Mittel zum Stimulieren von NK-Zellen anwendbar ist.

31. Zusammensetzung oder Mittel gemäß Anspruch 29 oder 30, die als Mittel zum Stimulieren der endogenen Produktion von Interleukin-2 anwendbar ist.

32. Zusammensetzung oder Mittel gemäß Anspruch 24 bis 27, die als schmerzstillendes Mittel anwendbar ist.

33. Zusammensetzung oder Mittel gemäß Anspruch 24 bis 27, die als Appetitzügler anwendbar ist.
